# EUROPEAN PATENT APPLICATION

(11) **EP 2 018 842 A1**
(43) Date of publication of application: **28.01.2009**
(21) Application number: 07014676.6
(22) Date of filing: 26.07.2007
(51) Int. Cl.: A61K 8/34, A61K 8/41, A61K 8/898, A61Q 5/06, A61Q 5/12

(54) **Conditioning and colouring composition for hair**

(71) Applicant: KPSS-Kao Professional Salon Services GmbH, 64297 Darmstadt (DE)
(72) Inventor: Bistram, Vera, 64683 Einhausen (DE)

(57) **Abstract**

The present invention concerns a conditioning and colouring composition for hair with optimum conditioning effects especially shine, softness and manageability.

It has surprisingly been found out that a hair conditioning composition comprising at least one oil soluble dye and at least one polypropylene glycol compound according to the formula 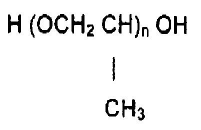 wherein n is a number in the range of 2 to 50, and
at least one cationic surfactant of the following general formula 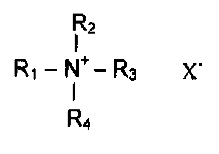 wherein R₁ is an saturated or unsaturated alkyl chain with 12 to 22 C atoms, or

R₅ CO NH (CH₂)ₙ

where R₅ is saturated or unsaturated, alkyl chain with 7-21 C atoms and n has value of 1 - 4, or

R₆ CO O (CH₂)n

where R₆ is saturated or unsaturated, branched or non-branched alkyl chain with 7-21 C atoms and n has value of 1 - 4, and
R₂, R₃ and R₄ are same or different and independent from each other an alkyl group with 1 to 4 C atoms, and X is an anion such as chloride, bromide, methosulfate
and/or at least one silicone compound with at least one quaternary ammonium group, conditions and enhances colour and shine of hair excellently.

## Description

The present invention concerns a conditioning and colouring composition for hair with optimum conditioning effects especially shine, softness and manageability.

Coloring and conditioning compositions have been known for years and have been proven to be very successful on the market.

Such compositions customarily comprise hair coloring direct dyestuffs together with the at least one hair conditioning compound, especially of cationic types.

Although these products are proven as such to be satisfying consumer needs, it is still desirable to improve their efficiency especially in terms of hair shine, softness and manageability together with good colour enhancing ability, and certainly with other hair conditioning properties in particular with regard to volume and body, and combability.

Thus, the object of the present invention is providing a conditioning composition showing excellent colour enhancing and conditioning abilities together with excellent shine enhancing effect, improving excellently manageability and softness of hair, at the same time having optimum conditioning effects on hair, which satisfies consumer expectations in terms of combability, volume and body, and nice feeling on touching hair.

It has surprisingly been found out that a hair conditioning composition comprising at least one oil soluble dye and at least one polypropylene glycol compound according to the formula 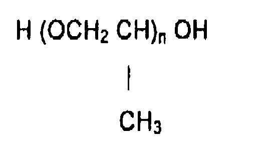 wherein n is a number in the range of 2 to 50, and
at least one cationic surfactant of the following general formula 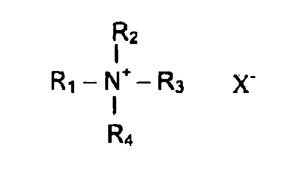 wherein R₁ is an saturated or unsaturated alkyl chain with 12 to 22 C atoms, or

R₅ CO NH (CH₂)ₙ

where R₅ is saturated or unsaturated, alkyl chain with 7-21 C atoms and n has value of 1-4, or

R₆ CO O (CH₂)ₙ

where R₆ is saturated or unsaturated, branched or non-branched alkyl chain with 7-21 C atoms and n has value of 1 - 4, and
R₂, R₃ and R₄ are same or different and independent from each other an alkyl group with 1 to 4 C atoms, and X is an anion such as chloride, bromide, methosulfate and/or
at least one silicone compound with at least one quaternary ammonium group, conditions and enhances colour and shine of hair excellently,

Further object of the present invention is method of conditioning and colour enhancing wherein a composition as disclosed in the above paragraph is applied onto hair and after massaging into hair and processing 1 to 30 min rinsed off with water.

Further object of the present invention is the use of the composition disclosed in the above paragraph for conditioning and colour enhancing hair.

Further object of the present invention is method of producing conditioning and colour enhancing composition wherein oil soluble dye is dissolve in at least one polypropylene glycol and mixed with at least one cationic surfactant and/or silicone compound with at least one quaternary ammonium group subsequently added to the rest of the composition.

With the term oil soluble it is meant that the dyestuff is soluble in the selected polypropylene glycol at the concentrations used in the formulations at room temperature or at temperatures where the composition is produced. Such temperatures may vary from room temperature to approximately 80°C.

Conditioning and colour enhancing compositions of the present invention comprise at least oil soluble dye. In principal any oil soluble dye is suitable for the purpose of the present invention. Non-limiting examples to suitable ones are with their colour index numbers Cl 12100, Cl 12140, Cl 12704, Cl 12740Cl 26100, Cl 26105, Cl 45396, Cl 45410, Cl 45425, Cl 47000, Cl 60725, Cl 60724, Cl 61520 and Cl 61565.

Concentration of oil soluble dyes in the compositions of the present invention varies in the range of 0.001 to 1%, preferably 0.005 to 0.75, more preferably 0.01 to 0.5 and most preferably 0.05 to 0.25% by weight calculated to total composition.

Cleansing and colour enhancing composition of the present invention comprise at least one polypropylene glycol according to the following general formula 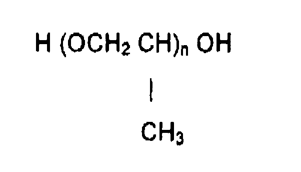 wherein n is a number between 2 and 50 preferably 2 and 30 more preferably 2 and 20.

Non-limiting examples are dipropylene glycol, PPG-3, PPG-9, PPG-12, PPG 15, PPG-16, PPG 20. Most preferred is dipropyleneglycol.

Concentration of the polypropylene glycol in the composition of the present invention varies between 0.1 and 20%, preferably 0.25 and 15% and more preferably 0.5 and 10%, and most preferably 0.75 to 5% by weight, calculated to total composition.

Compositions of the present invention comprise at least one mono alkyl quaternary ammonium surfactant with the given general formula above and/or a silicone compound with at least one quaternary ammonium group.

Non-limiting examples to mono alkyl quaternary ammonium surfactant are cetrimonium chloride, steartrimonium chloride, behentrimonium chloride, cocotrimonium methosulfate, cocotrimonium chloride, cocamidoproplyethyldimonium methosulfate, cocamidopropyltrimonium chloride and stearamidopropylethyldimonium methosulfate. Preferred are cetrimonium chloride, steartrimonium chloride and behentrimonium chloride. Most preferred is cetrimonium chloride.

Concentration of mono alkyl quaternary ammonium surfactant is in the range of 0.1 to 3%, preferably 0.2 to 2% more preferably 0.25 to 1.5% and most preferably 0.3 to 1% by weight calculated to total composition.

Silicone compounds comprising at least one quaternary ammonium group are comprised in the same concentration ranges as given for mono alkyl quaternary ammonium compounds.

As a rule any silicone compound carrying at least one quaternary ammonium group is suitable for the purpose of the present invention. Preferably the silicone compound comprises two quaternary ammonium groups.

Suitable examples are those known with the CTFA name silicone Quaternium-1 to 21 and Quaternium 80. The former ones include single quaternary ammonium group and the latter two quaternary ammonium groups.

Most preferred silicone compound with quaternary ammonium group is Quaternium-80 available from Degussa under the trade name Abil Quat.

The pH of the compositions according to the present invention is in the range of 2 to 6, preferably in the range of 2.5 to 5.0, more preferably 2.5 to 4.5, most preferably 2.5 to 4.00.

The pH of the compositions is adjusted with hydroxycarboxilic acids and/or dicarboxylic acids. In those cases where selected hydroxycarboxylic acid and/or dicarboxylic acid concentration is not enough to reach the selected pH, other organic and inorganic acids may as well be used to adjust pH to the required value. The hydroxycarboxilic acids useful in the compositions of the present invention are lactic acid, glycolic acid, hydroxyacrylic acid, glyceric acid, malic acid and tartaric acid and of the dicarboxylic acids are malonic acid, succinic acid, glutaric acid, adipic acid, maleic acid, fumaric acid and phtalic acid.

According to the invention, colour enhancing cleansing composition can further comprise at least one additional direct acting hair dye. Suitable dyes are anionic, cationic and nonionic nitro dyes.

In the preferred form compositions of the present invention comprise at least one cationic direct dye. Suitable cationic dyestuffs are in principal those available on the market for hair colouring applications. Some examples to those are:

Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Orange 31, Basic Red 2, Basic Red 12, Basic Red 51, Basic Red 22, Basic Red 76, Basic Violet 1, Basic Violet 2, Basic Violet 3, Basic Violet 10, Basic Violet 14, Basic Yellow 57 and Basic Yellow 87. For this purpose, special reference is made to the PCT application WO 95/15144 of Ciba-Geigy AG. All of the cationic dyes disclosed in the referred publication are suitable for the purpose of the present invention.

Anionic dyes may as well be used alone or in combination with cationic direct dyes. The suitable ones are. Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92. Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium. It should be noted that colouring effect of tose anionic dyes are much lower compared to the oil soluble ones and cationic dyes, and therefore they are used for adjusting the colour of the composition, bu not hair.
Additionally, the coloring compositions of the present invention can comprise neutral dyes (HC dyes), so called nitro dyes either alone or in addition to the cationic direct dyes.

Some examples to those are: HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15,2-Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Dyes in addition to the oil soluble dye can be included into the compositions of the present invention at a concentration of 0.0001 to 1%, preferably 0.0001 to 0.75% and more preferably 0.0001 to 0.5% by weight, calculated to total aqueous composition.

Colouring and conditioning compositions of the present invention comprise at least one hair-conditioning compound. Suitable ones are those of cationic compounds, especially cationic polymers and cationic surfactants. Oily substances and non-ionic substances can as well be suitably used as conditioners in the compositions of the present invention.

Suitable cationic polymers are those of best known with their CTFA category name Polyquaternium. Typical examples of those Polyquaternium 6, Polyquaternium 7, Polyquaternium 10, Polyquaternium 11, Polyquaternium 16, Polyquaternium 22 and Polyquaternium 28.

As well those polymers known with their CTFA category name Quaternium are suitable. Those are for example Quaternium-8, Quaternium-14, Quaternium-15, Quaternium-18, Quaternium-22, Quaternium-24, Quaternium-26, Quaternium-27, Quaternium-30, Quatemium-33, Quaternium-53, Quaternium-60, Quaternium-61, Quaternium-72, Quaternium-78, Quaternium-80, Quaternium-81, Quaternium-81, Quaternium-82, Quaternium-83 and Quaternium-84.

It has further been found out that especially those of cationic cellulose type polymers known as Polymer JR type from Amerchol such as Polyquaternium 10 or cationic guar gum known with trade name Jaguar from Rhône-Poulenc and chemically for example Guar hydroxypropyl trimonium chloride, are preferred ones. Furthermore, chitosan and chitin can also be included in the compositions as cationic natural polymers. In this context reference is also made to the cationic polymers disclosed in DE 25 21 960, 28 11 010, 30 44 738 and 32 17 059, as well as to the products described in EP-A 337 354 on pages 3 to 7. It is also possible to use mixtures of various cationic polymers.

The most preferred cationic polymers are those of cationic cellulose derivatives, cationic guar gum derivatives, polyquaternium 6 and polyquaternium 7.

The cationic polymers also include the quaternized products of graft polymers from organopolysiloxanes and polyethyl oxazolines described in EP-A 524 612 and EP-A 640 643.

Typical concentration range for cationic polymers is 0.01 - 5% by weight, preferably 0.01 - 3.5% by weight, more preferably 0.05 - 2.5% and most preferably 0.05 - 1.5% by weight calculated to the total composition.

In one of the preferred from of the present invention, conditioning compositions comprise at least one additional cationic surfactant other than the mono alkyl cationic surfactants mentioned above as conditioning agent. Suitable cationic surfactants are presented with the general formula presented above (page 2)
where R₁ and R₂ is a saturated or unsaturated, branched or non-branched alkyl chain with 8-22 C atoms or

R₅ CO NH (CH₂)ₙ

where R₅ is saturated or unsaturated, branched or non-branched alkyl chain with 7-21 C atoms and n has value of 1 - 4,
or

R₆ CO O (CH₂)ₙ

where R₆ is saturated or unsaturated, branched or non-branched alkyl chain with 7-21 C atoms and n has value of 1 - 4, and

R₃ and R₄ are lower alkyl chain with 1 to 4 carbon atoms, and X is anion such as chloride, bromide, methosulfate.

Typical non-limiting examples of those ingredients are dicetyl diimethly ammonium chloride, distearyl diimonium chloride, dioleoylethyl dimethyl ammonium methosulfate, dioleoylethyl hydroxyethylmonium methosulfate.

From the above quaternary ammonium compounds disclosed with the general formula, especially to mention are those compounds known **per se** and are on the market, for example, under the trade names "Schercoquat^{®}", "Dehyquart^{®} F30" and "Tetranyl^{®}". Use of these compounds, the so-called "esterquats", in hair care compositions is described, for example, in WO-A 93/107 48, WO-A 92/068 99 and WO-A 94/166 77. wherein, however, there is no reference made to the combinations according to the present invention and the advantageous properties thereof.

Again from the above quaternary ammonium compounds disclosed with the general formula, especially to metion are these compounds are known **per se** and on the market, for example, under the trade name "INCROQUAT^{®} HO" or "OCS". Those compounds are known with a general ingredient category under "amidoquat" in the cosmetic industry.

Amido amines may as well be used as a conditioning cationic surfactant in the compositions of the present invention. Typical non-limiting example is stearamidopropylamine known with a trade name Tego Amid S18 from Goldschmidt.

Typical concentration range for cationic surfactants other than those of mono alkyl quaternary ammonium surfactants is 0.01 - 5% by weight, preferably 0.01 - 3.5% by weight, more preferably 0.05 - 2.5% and most preferably 0.05 - 1.5% by weight calculated to the total composition.

It is as well the preferred embodiment of the invention that the cationic surfactants as conditioners and cationic polymers used in combination at any ratio.

Oily substances are selected from such as silicone oils, either volatile or non-volatile, natural and synthetic oils. Among silicone oils those can be added to the compositions include dimethicone, amodimethicone (a cationic silicone), dimethiconol, polydimethylsiloxane, DC fluid ranges from Dow Corning, natural oils such as olive oil, almond oil, avocado oil, weizenkeim oil, ricinus oil and the synthetic oils, such as mineral oil, isopropyl myristate, palmitate, stearate and isostearate, oleyl oleate, isocetyl stearate, hexyl laurate, dibutyl adipate, dioctyl adipate, myristyl myristate and oleyl erucate.

Non-ionic conditioning agents may be polyols such as glycerin, glycol and derivatives, polyethyleneglycoles known with trade names Carbowax PEG from Union Carbide and Polyox WSR range from Amerchol, polyglycerin polyethyleneglycol mono or di fatty acid esters having general formula

R₇ CO (O CH₂CH₂)ₙ OH

R₇ CO (O CH₂CH₂)ₙ O OC R₈

where R₇ and R₈ are independent from each other saturated, unsaturated or branched or non-branched alkyl chain with 7 to 21 C atoms and n is typically 2 -100.

The compositions according to the invention may also comprise further conditioning substances such as protein hydrolyzates and polypeptides, e.g., keratin hydrolyzates, collagen hydrolyzates of the type "Nutrilan^{R}" or elastin hydrolyzates, as well as also in particular plant protein hydrolyzates, optionally, cationized protein hydrolyzates, e.g., "Gluadin^{R}"_{.}

Further conditioning additives are hair conditioning and/or styling polymers. These may be nonionic polymers, preferably alcohol- and/or water-soluble vinyl pyrrolidone polymers, such as a vinyl pyrrolidone homopolymers or copolymers, in particular with vinyl acetate. Useful vinyl pyrrolidone polymers are, e.g., those known by the trade name "Luviskol®", for example, the homopolymers "Luviskol® K 30, K 60 and K 90", as well as the water-or alcohol-soluble copolymers from vinyl pyrrolidone and vinyl acetate, distributed by BASF AG under the trade name "Luviskol® VA 55 respectively VA 64", Further possible nonionic polymers are vinyl pyrrolidone/vinyl acetate/vinyl propionate copolymers such as "Luviskol® VAP 343", vinyl pyrrolidone/(meth)acrylic acid ester copolymers, as well as chitosan derivatives.

Amphoteric polymers are found to be useful in conditioning and colouring composition of the present invention. They are incorporated alone or in admixture with at least one additional cationic, nonionic or anionic polymer, particularly copolymers of N-octyl acrylamide, (meth)acrylic acid and tert-butyl aminoethyl methacrylate of the type "Amphomer®"; copolymers from methacryl oylethyl betaine and alkyl methacrylates of the type "Yukaformer®", e.g., the butyl methacrylate copolymer "Yukaformer® Am75"; copolymers from monomers containing carboxyl groups and sulfonic groups, e.g., (meth)acrylic acid and itaconic acid, with monomers such as mono- or dialkyl amino alkyl(meth)acrylates or mono- or dialkyl aminoalkyl (meth)acrylamides containing basic groups, in particular amino groups; copolymers from N-octyl acrylamide, methyl methacrylate, hydroxypropyl methacrylate, N-tert,-butyl aminoethyl methacrylate and acrylic acid, as well as the copolymers known from US-A 3,927,199, are applicable.

Colouring and conditioning compositions of the present invention can be in the form of emulsions, solutions, gels and dispersions. In the case that solutions and/or gels forms are prefered the appearance can be either with a transparent or opaque. Transparency of the composition is judged by naked eye in a transparent bottle with a thickness not more than 5 cm. As a product form, foam is as well suited when packed into a pressurized can or delivered through a pump-foamer (non-aerosol). In the case that an aerosol foam preparation is preferred, propellant gas must be added to the formulation. The suitable propellant gasses are carbondioxide, dimethylether and alkanes such as butane, propane, isobutane or their mixtures.

Compositions of the present invention preferably comprise at least one thickening agent such as anionic polymers such as acrylate homo or copolymers, nonionic polymers such as cellulose derivatives such as hydroxyethyl cellulose, hydroxypropyl cellulose, xanthan gum, guar gum, cationic polymers such as cationic derivatives of cellulosic polymer or guar gum. It may as well be above mentioned thickeners are used in mixture with paying attention to the compatibility with each other. Concentration of thickeners in total can vary between 0.1 and 2.5%, preferably 0.2 and 2% and morepreferably 0.25 to 1.5% by weight calculated to total composition.

The emulsion type of colouring conditioners comprise additionally at least one fatty alcohol of the following formula

R₉-OH

where R₉ is a saturated or unsaturated, branched or non-branched fatty acyl chain with 8 - 24 C atoms. Concentration of fatty alcohols is usually less than 20%, preferably less than 15% by weight calculated to total composition. Typical examples to the most useful fatty alcohols are myristyl alcohol, palmityl alcohol, cetyl alcohol, stearyl alcohol, behenyl alcohol and their mixtures. As a mixed fatty alcohol the mostly used one is the cetearyl alcohol as well preferred in the compositions of the present invention.

The composition of the present invention can comprise surface-active substances as emulsifiers other than cationic surfactants disclosed above especially when an emulsion is preferred. These can be anionic and/or nonionic and/or amphoteric or zwitterionic and/or their mixtures incorporated at a concentration ranging between 0.1 - 10 %, preferably 0.1 - 7.5% and more preferably 0.1 - 5% by weight calculated to the total composition. Preferred emulsifiers are of non-ionic surfactants in addition to the cationic surfactants disclosed above. The anionic ones are as a rule not preferred and if their presence is desirable because of any reason, those should form the very minor part, as electrostatic interactions with the cationic material especially dyes and cationic conditioners either surfactants or polymers can disturb the stability of those conditioners as well prevent colouring effect. Zwitterionic ones are also the ones preferred to lesser extent.

Suitable preferred nonionic surfactants useful in the compositions according to invention are C₁₀-C₂₂-fatty alcohol ethoxylates. Especially suited are C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16":

The average degree of ethoxylation thereby ranges between about 2.5 and about 25, preferably about 10 and about 20.

Further suitable nonionic surfactants according to the invention are alkyl polyglucosides of the general formula

R₁₀-O-(R₁₁O)ₙ-Zₓ,

wherein R₁₀ is an alkyl group with 8 to 18 carbon atoms, R₁₁ is an ethylene or propylene group, Z is a saccharide group with 5 to 6 carbon atoms, n is a number from 0 to 10 and x is a number between 1 and 5.

Further nonionic surfactants are, for example, long-chain fatty acid mono- and dialkanolamides, such as coco fatty acid monoethanolamide and myristic fatty acid monoethanolamide.

Further useful nonionic surfactants are, for example, the various sorbitan esters, such as polyethylene glycol sorbitan stearic acid ester, fatty acid polyglycol esters or polycondensates of ethyleneoxide and propyleneoxide, as they are on the market, for example, under the trade name "Pluronics^{R}", as well as fatty alcohol ethoxylates.

Further suitable nonionic surfactants are amineoxides. Such amineoxides are state of the art, for example C₁₂-C₁₈- alkyl dimethyl amineoxides such as lauryl dimethyl amineoxide, C₁₂-C₁₈- alkyl amidopropyl or -ethyl amineoxides, C₁₂-C₁₈ -alkyl di(hydroxyethyl) or (hydroxypropyl) amineoxides, or also amineoxides with ethyleneoxide and/or propyleneoxide groups in the alkyl chain. Such amineoxides are on the market, for example, under the trade names "Ammonyx^{®}", "Aromox^{®}" or "Genaminox^{®}".

As further surfactant component, the compositions according to the invention can also contain amphoteric or zwitterionic surfactants. Useful as such are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate have also proven suitable.

Anionic surfactants are as a rule preferred to a lesser extend. Some of them are to mention those of sulfate, sulfonate, carboxylate and alkyl phosphate types. Examples are the known C₁₀-C₁₈-alkyl sulfates, and in particular the respective ether sulfates, for example, C₁₂-C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates.

Additional anionic surfactants are α-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof as well as alkyl amido polyether carboxylic acids. Such products have been known for some time and are on the market, for example, under the trade name "AKYPO^{®}" and "AKYPO-SOFT^{®}".

The others to mention are C₈-C₂₀-acyl isethionates, as well as sulfofatty acids and the esters thereof.

Further anionic surfactants are also C₈-C₂₂-acyl aminocarboxylic acids or the water-soluble salts thereof. Especially preferred is N-lauroyl glutamate, in particular as sodium salt, as well as, for example, N-lauroyl sarcosinate, N-C₁₂-C₁₈-acyl asparaginic acid, N-myristoyl sarcosinate, N-oleoyl sarcosinate, N-lauroyl methylalanine, N-lauroyl lysine and N-lauroyl aminopropyl glycine, preferably in form of the water-soluble alkali or ammonium, in particular the sodium salts thereof, preferably in admixture with the above-named anionic surfactants.

In another preferred form of the invention, compositions comprise at least one organic solvent. Suitable ones are ethanol, propanol, isopropanol, benzyl alcohol, benzyloxyethanol, ethoxydiglycol, alkylene carbonates such as ethylene carbonate and propylene carbonate, phenoxyethanol, butanol, isobutanol, cyclohexane, cyclohexanol, hexyleneglycol, ethylenecarbonate, propyleneglycol, ethyleneglycol monoethylether, ethylene glycol monobutyl ether, ethylene glycol monophenyl ether, 1-phenylethylalcohol, 2-phenylethylalcohol, o-methoxyphenol. The most preferred ones are benzyloxyethanol and polypropylene glycols. Concentration of organic solvents in the compositions should not exceed 20% by weight, preferably in the range of 0.1 to 15%, more preferably 0.2 to 10% and most preferably 0.5 to 5% by weight calculated to total composition.

The viscosity of the compositions depends on the type of application according to the invention. Emulsion type of compositions have the viscosity in the range of between about 1000 and about 50,000 mPa.s at 20°C, measured according to Brookfield or Höppler at a shear rate of 10 sec⁻¹. Whereas compositions dispensed form an aerosol and/or pump foamer should preferably be very liquid, i.e. viscosity values not more than approximately 500 mPa.s measured as given above are not appropriate.

Compositions of the present invention are used especially after shampooing and can be applied to hair either as a leave-in or as a rinse off compositions.

Furthermore, compositions of the present invention can comprise all substances customarily found in hair conditioning preparations.

Examples of such substances are natural plant extracts, complexing agents, preservatives, fragrances, moisturizers, UV filters, etc.

Natural plant extracts are incorporated usually in an amount of about 0.01 % to about 10 %, preferably 0.05 % to 7.5 %, in particular 0.1 % to 5 % by weight, calculated as dry residue thereof to the total composition. Suitable aqueous (e.g. steam-distilled) alcoholic or hydro-alcoholic plant extracts known **per se** are in particular extracts from leaves, fruits, blossoms, roots, rinds or stems of aloe, pineapple, artichoke, arnica, avocado, valerian, bamboo, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, cocoanut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc.

Suitable trade products are, for example, the various "Extrapon^{®}" products, "Herbasol^{R} ", "Sedaplant^{R}" and "Hexaplant^{R}". Extracts and the preparation thereof are also described in "Hagers Handbuch der pharmazeutischen Praxis", 4^{th} Ed..

Compositions of the present invention can comprise UV filters for protection of hair from environmental influences such as loss of elasticity, loss of hair colour (bleaching effect of sun light). The UV-absorbing substance is preferably selected from the following compounds: 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2.4-dihydroxybenzophenone, 2.2'.4.4'-tetrahydroxybenzophenone, 2-hydroxy-4-methoxybenzophenone and its 5-sulfonic acid or the sodium salt thereof, 2.2'-dihydroxy-4.4'-dimethoxybenzophenone, 2-hydroxy-5-chlorobenzophenone, 2.2'-dihydroxy-4-methoxybenzophenone, 2,2-dihydroxy-4.4'-dimethoxy-5.5'-disulfobenzo-phenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzyl-idenebornane-2-one and the salts thereof and/or 3-(4'-methyl benzylidene)-DL-campher.

The preferred amount of the UV-absorber ranges from about 0.01 % to 2.5%, more preferably from 0.05 % to 1 % by weight, calculated to the total composition.

Compositions of the present inventione can further comprise at least one ubichinone according to formula 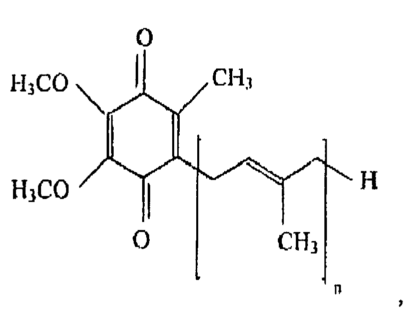 wherein n is 1 to 10. Preferred compound of the formula is ubiquinone 10 wherein n is 10 which is also known as Coenzyme Q10.

Concentration of at least one ubiquinone is in the range of 0.001 to 1% by weight, preferably not more than 0.75% by weight and more preferably not more than 0.5% by weight, calculated to total composition.

The compositions of the present invention can comprise hair-restructuring agent such as cetyl PG hydroxyethyl palmitamide.

Other preferred hair restructuring agents are fatty acids with 10 to 24 carbon atoms and especially with 16 to 24 carbon atoms.

Sterols,especially the phytosterols, are as well preferred hair restructuring agents as disclosed in the above mentioned german patent. Especially preferred ones are of plant origin for example ergosterol, sitosterol, stigmasterol, fucosterol, brassicasterol, fungisterol, campesterol, zymosterol, ascosterol, cerevisterol, episterol, faecosterol, spinasterol. Among those phytosterols, the ones found in "Avocadin" which is the unsaponified fraction of the avocado oil is more preferred.

The concentration of ceramide in the compositions of the present invention can be in the range of 0.01 to 2% and especially 0.01 to 1% by weight calculated to the total weight of the composition. The fatty acids may be contained at a level of 0.01 to 2.5% and epecially 0.01 to 1% by weight calculated to the total weight of the composition Phytosterol concentration of the conditioners is less than 1% and preferably less than 0.5% by weight calculated to the total weight of the composition. It should be noted without limiting the use of those ingredients the effect of those hair restructuring ingredients is especially elevated when used in combination with penetration enhancers.

The moisturizing agents are selected from panthenol, polyols, such as glycerol, polyethylene glycols with molecular weight 200 to 20,000. The moisturizing ingredients can be included in the conditioner compositions at a concentration range of 0.01 - 2.5% by weight calculated to the total composition.

The sequestering agents are selected from polycarboxy acids. The preferred one is ethylene diamine tetraacetic acid, EDTA. Typical useful concentration range for sequestering agents is of 0.01 - 2.5% by weight calculated to the total composition.

In an another preferred embodiment of the present invention is that the composition disclosed ghere are used in combination with a cleansing composition having similar pH values and preferably comprising hydroxycarboxylic acid and/or dicarboxylic acid. In other words, the hair is cleansed firs with a composition comprising at least one cleansing surfactant and at least one hydroxycarboxylic acid and/or dicarboxylic acid subsequently treated with compositions of the present invention. This has resulted in that the all the effects especially shine and manageability is observed at elevated levels and as well as shorter usage period.

The following examples illustrate the invention.

### Example 1

| | % by weight |
|---|---|
| Hydroxyethylcellulose | 0.8 |
| Stearyltrimethylammoniumchlorid | 3.0 |
| Dipropyleneglycol | 2.0 |
| Cl 60725 | 0.1 |
| Fragrance, preservative | q.s. |
| Malic acid | q.s. to pH 3.5 |
| Wasser | q.s. 100.0 |

Above composition was prepared by dissolving dyestuff in dipropylene glycol and mixing cationic surfactant into the solution. Afterwards the mixture was combined with hydroxyethylcellulose solution and other ingredients were added and finally pH was adjusted.

Above composition was used on a gray fine hair as a conditioner. After shampooing appropriate amount was applied onto hair left on the hair for about 5 min and rinsed off with water. After drying hair, shiny, silver gray hair was observed which had significantly less yellow.

### Example 2

| | % by weight |
|---|---|
| Hydroxyethylcellulose | 0.8 |
| Dimethicone copolyol | 1.0 |
| Cetrimonium chloride | 2.5 |
| Dipropyleneglycol | 3.0 |
| Cl 60725 | 0.08 |
| Basic blue 99 | 0.05 |
| Fragrance, preservative | q.s. |
| Malic acid | q.s. to pH 3.5 |
| Wasser | q.s. 100.0 |

Above composition was prepared in the same way as in example 1 except Basic blue 99 was dissolved in a small portion of water and added to the cellulose solution.

Above composition was tested on a bleached hair. Above conditioner was applied after shampooing hair and rinsed off after 10 min processing at room temperature. It was observed that hair feels soft and combable and has less yellowish appearance after use of the above conditioner. The effect became more and more visible after repeated usage.

### Example 3

| | |
|---|---|
| Cetylstearylalcohol | 5.0 (% by weight) |
| Cetrimonium chloride | 3.0 |
| Dipropylene glycol | 2.5 |
| Cl 60725 | 0.125 |
| Basic red 76 | 0.03 |
| Fragrance, preservative | q.s. |
| Lactic acid | q.s. pH 4.0 |
| Wasser | ad 100.0 |

Similar results were observed as in the above examples.

Above composition is an emulsion composition and prepared by dissolving dyestuff in dipropyleneglycol and it was combined with half of the cetrimonium chloride and added to the in water emulsified fatty alcohol cationic surfactant mixture. The preparation was made at 70°C. After cooling the other ingredients were added.

### Example 4

| | |
|---|---|
| Cetylstearylalcohol | 5.0 (% by weight) |
| Cetrimonium chloride | 1.75 |
| Quaternium-80 | 1.50 |
| Dipropylene glycol | 2.5 |
| Cl 60725 | 0.125 |
| Fragrance, preservative | q.s. |
| Lactic acid | q.s. pH 4.0 |
| Wasser | ad 100.0 |

Excellent antiyellow effect was observed on bleached and gray hair.

### Example 5

| | |
|---|---|
| **Foam conditioner** | |
| Quaternium-80 | 0.2 (Gew.-%) |
| Polyquaternium-11 | 0.7 |
| PEG-160-hydrogenated ricinus oil | 0,5 |
| Fragrance, preservative | q.s. |
| PPG-3 | 2.5 |
| Basic red 76 | 0.05 |
| Cl 60725 | 0.15 |
| Basic blue 99 | 0.04 |
| Citric acid | q.s. to 3.2 |
| Wasser | ad 100.0 |

The composition is suitable for leave-in and rinse off. In leave-in application, amount used is obviously less than in the case of a rinse of application. It has an excellent anti yeloow effect on gray and bleached hair. It can also be used on blonde hair. The composition is packed into an aerosol can with 90/10 ratio, by weight, liquid composition to propellant. As propellant propane, butane mixture is used.

### Example 6

| | |
|---|---|
| Cetylstearylalcohol | 4.0 (% by weight) |
| Cetrimoniumchloride | 2.0 |
| Panthenol | 0,4 |
| Dimethicone | 0.75 |
| Hydroxypropyl Guar Hydroxypropyltrimonium | |
| Chloride | 1.0 |
| Avocado extract | 0.5 |
| Fragrance, preservative | q.s. |
| Malic acid | q.s to pH 3.5 |
| Basic red 51 | 0.035 |
| Cl 60725 | 0.125 |
| PPG-3 | 2.5 |
| Wasser | ad 100.0 |

Above composition delivers excellent antiyellow effect on gray and bleached hair.

### Example 7

| | |
|---|---|
| Cetylstearylalcohol | 5.0 (% by weight) |
| Steartrimonium Chloride | 1.5 |
| Dioleoylethyl dimethyl ammonium methosulfate | 0.4 |
| Mineral Oil( Paraffinum liquidum) | 0.5 |
| Isopropyl Myrisate | 0.3 |
| Bamboo extract | 0.3 |
| Dipropylen glycol | 3.0 |
| Ubichinone-10 | 0.05 |
| Fragrance, preservative | q.s. |
| Malic acid | q.s. to pH 3.5 |
| Cl 60725 | 0.09 |
| Acid red 52 | 0.002 |
| Wasser | add 100.0 |

Above composition has excellent conditioning and anti-yellow effect on multiple bleached hair.

It is also possible to use the above composition as leave in composition on any type of the hair for excellent anti-yellow effect.

### Example 8

| | |
|---|---|
| Cetylstearylalcohol | 5.0 (% by weight) |
| Steartrimonium Chloride | 1.5 |
| Quaternium-80 | 1.0 |
| Mineral Oil( Paraffinum liquidum) | 0.5 |
| Isopropyl Myrisate | 0.3 |
| Dipropylene glycol | 3.0 |
| Fragrance, preservative | q.s. |
| Lactic acid | q.s. to pH 3.8 |
| Basic Blue 99 | 0.005 |
| Cl 60725 | 0.09 |
| HC red 3 | 0.005 |
| Wasser | add 100.0 |

The composition has a superior performance on gray hair in terms of colour equalizing and as well as show anti-yellow effect on blond and bleached hair.

## Claims

1. Conditioning composition for hair **characterised in that** it comprises at least one at least one oil soluble dye and at least one polypropylene glycol compound according to the formula 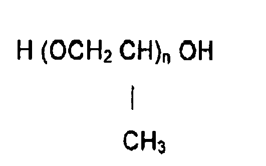 wherein n is a number in the range of 2 to 50, and
at least one cationic surfactant of the following general formula 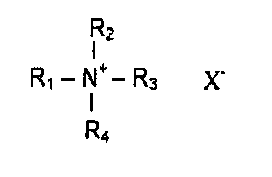 wherein R₁ is an saturated or unsaturated alkyl chain with 12 to 22 C atoms, or
R₅ CO NH (CH₂)ₙ
where R₅ is saturated or unsaturated, alkyl chain with 7-21 C atoms and n has value of 1 - 4,
or
R₆ CO O (CH₂)ₙ
where R₆ is saturated or unsaturated, branched or non-branched alkyl chain with 7-21 C atoms and n has value of 1 - 4, and
R2, R3 and R4 are same or different and independent from each other an alkyl group with 1 to 4 C atoms, and X is an anion such as chloride, bromide, methosuffate and/or
at least one silicone compound with at least one quaternary ammonium group.

2. Composition according to claim 1 **characterized in that** oil soluble dyes are selected from Cl 12100, Cl 12140, Cl 12700, Cl 12740Cl 26100, Cl 26105, Cl 45396, Cl 45410, Cl 45425, Cl 47000, Cl 60725, Cl 60724, Cl 81520 and Cl 61565.

3. Composition according to claims 1 and 2 **characterized in that** it comprises polypropylene glycol selected from dipropylene glycol, PPG-3, PPG-9, PPG-12, PPG 15, PPG-16, PPG 20 at a concentration of 0.1 to 10% by weight, calculated to total composition.

4. Composition according to any of the preceding claims **characterised in that** it comprises mono alkyl quaternary ammonium surfactant and/or silicone compound with at least one quaternary ammonium group at a concentration of 0.1 to 3 % by weight calculated to total composition.

5. Composition according to claim 4 **characterised in that** mono alkyl quaternary ammonium surfactant is cetrimonium chloride.

6. Composition according to claim 4 **characterised in that** silicone compound with at least one quaternary ammonium group is Quaternium-80.

7. Composition according to any of the preceding claims **characterised in that** it has a ph in the range of 2 to 6.

8. Composition according to any of them preceding claims **characterised in that** it comprises at least one additional direct dye selected from anionic, cationic and non-ionic nitro dye.

9. Composition according to claim 8 **characterised in that** it comprises at least one cationic direct dye at a concentration of 0.0001 to 1% by weight, calculated to total composition.

10. Composition according to any of the preceding claims **characterised in that** it comprises conditioning agents selected from oily substances, non-ionic substances, cationic polymers or their mixture.

11. Composition according to any of the preceding claims **characterised in that** it comprises an additional cationic surfactant wherein R₁ and R₂ is a saturated or unsaturated, branched or non-branched alkyl chain with 8-22 C atoms or
R₅ CO NH (CH₂)ₙ
where R₅ is saturated or unsaturated, branched or non-branched alkyl chain with 7-21 C atoms and n has value of 1 - 4, or
R₆ CO O (CH₂)ₙ
where R₆ is saturated or unsaturated, branched or non-branched alkyl chain with 7-21 C atoms and n has value of 1 - 4, and R₃ and R₄ are lower alkyl chain with 1 to 4 carbon atoms, and X is anion such as chloride, bromide, methosulfate.

12. Composition according to any of the preceding claims it comprises at least one fatty alcohol of the following formula
R₉-OH
where R₉ is a saturated or unsaturated, branched or non-branched fatty acyl chain with 8 - 24 C atoms.

13. Composition according to any of the preceding claims **characterized in that i**t comprises at least one surfactant.

14. Composition according to any of the preceding claims **characterised in that** it comprises at least one UV filter.

15. Composition according to any of the preceding claims **characterised in that** it comprises at least one organic solvent.

16. Composition according to any of the preceding claims **characterised in that** it comprises at least one ubichinone of the following formula 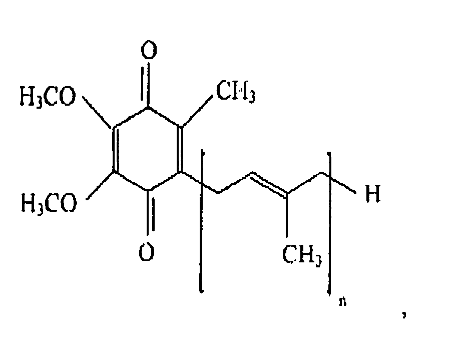 wherein n is 1 to 10.

17. Use of a composition according to claims 1 to 16 for conditioning and colour enhancing hair.

18. Method of producing conditioning and colour enhancing composition according to claims 1 to 16 wherein oil soluble dye is dissolved in at least one polypropylene glycol and mixed with at least one cationic surfactant and/or silicone compound With at least one quaternary ammonium group subsequently added to the remaining ingredients in the formulation.
